Europäisches Patentamt

European Patent Office

Office européen des brevets

(19)

(11) Veröffentlichungsnummer: **0 354 993**

**A2**

# (12) EUROPÄISCHE PATENTANMELDUNG

(21) Anmeldenummer: 89112553.6

(22) Anmeldetag: 10.07.89

(51) Int. Cl.⁴: **C07C 213/06** , **C07C 233/17** , **C07C 217/08**

(30) Priorität: 18.07.88 DE 3824304

(43) Veröffentlichungstag der Anmeldung:
21.02.90 Patentblatt 90/08

(84) Benannte Vertragsstaaten:
AT BE CH DE ES FR GB GR IT LI LU NL SE

(71) Anmelder: Henkel Kommanditgesellschaft auf Aktien
Postfach 1100 Henkelstrasse 67
D-4000 Düsseldorf 1(DE)

(72) Erfinder: Lange, Fritz, Dr.
Bühne 22
D-4300 Essen(DE)
Erfinder: Schlechtriem, Wolfgang
Viernheimer Weg 6
D-4000 Düsseldorf 1(DE)

(54) Verfahren zur Herstellung von Anlagerungsprodukten von Ethylenoxid und/oder Propylenoxid an Amine bzw. Amide.

(57) Ein Verfahren zur Herstellung von Anlagerungsprodukten von Ethylenoxid und/oder Propylenoxid an reaktionsfähige Wasserstoffatome aufweisende Amine bzw. Amide in molaren Verhältnissen von 1 : 1 bis 20 : 1 durch Umsetzung der Amine bzw. Amide mit Ethylenoxid und/oder Propylenoxid unter erhöhter Temperatur und überatmosphärischem Druck in Abwesenheit von Lösemitteln und in Anwesenheit katalytischer Mengen von Alkalimetallhydroxiden und/oder Alkalimetallalkoxiden ergibt hellfarbige Anlagerungsprodukte, wenn man mit erhöhten Katalysatorkonzentrationen in Mengen von 1,5 bis 5, insbesondere 2 bis 5 Gew.-%, bezogen auf das Endprodukt der Reaktion, arbeitet.

EP 0 354 993 A2

Verfahren zur Herstellung von Anlagerungsprodukten von Ethylenoxid und/oder Propylenoxid an Amine bzw. Amide

Die Erfindung betrifft ein Verfahren zur Herstellung von Anlagerungsprodukten von Ethylenoxid und/oder Propylenoxid an reaktionsfähige Wasserstoffatome aufweisende Amine bzw. Amide in molaren Verhältnissen von 1 : 1 bis 20 : 1 durch Umsetzung der Amine bzw. Amide mit Ethylenoxid und/oder Propylenoxid unter erhöhter Temperatur und überatmosphärischem Druck in Abwesenheit von Lösemitteln und in Anwesenheit katalytischer Mengen von Alkalimetallhydroxiden und/oder Alkalimetallalkoxiden.

Bei der Herstellung von Anlagerungsprodukten insbesondere von Ethylenoxid an Amine bzw. Amide in Verfahren der vorstehend genannten Art entstehen unter den Standardbedingungen dunkel gefärbte Produkte, wodurch die Einsatzmöglichkeiten derselben beschränkt sind.

Es ist bekannt, als Katalysatoren für die Herstellung der vorgenannten Anlagerungsprodukte Alkalimetallhydroxide oder Alkalimetallmethylate in Konzentrationen von 0,1 bis 0,5 Gew.-%, bezogen auf das Endprodukt der Anlagerungsreaktion, einzusetzen. Es wurde nun überraschenderweise gefunden, daß insbesondere bei der Herstellung von Anlagerungsprodukten des Ethylenoxids bzw. des Ethylenoxids und des Propylenoxids an die vorgenannten Amine bzw. Amide hellfarbige Anlagerungsprodukte erhalten werden, wenn man die Alkalimetallhydroxide und/oder Alkalimetallalkoxide in höheren

Konzentrationen, d.h. in einer Menge von 1,5 bis 5 Gew.-%, vorzugsweise 2 bis 5 Gew.-%, bezogen auf das Endprodukt der Reaktion, einsetzt.

Mit dem Verfahren der Erfindung lassen sich Anlagerungsprodukte ausschließlich von Ethylenoxid und gemischte Anlagerungsprodukte von Ethylenoxid und Propylenoxid (in random- oder block-Verteilung der Ethylenoxid- und Propylenoxideinheiten) an reaktionsfähige Wasserstoffatome aufweisende Amine bzw. Amide herstellen; das Verfahren der Erfindung ist jedoch auch zur Herstellung von Anlagerungsprodukten ausschließlich des Propylenoxids geeignet, wenngleich die erfindungsgemäß erzielten Vorteile bei der Propoxylierung nicht so deutlich in Erscheinung treten.

Als erfindungsgemäß einsetzbare Katalysatoren eignen sich insbesondere Lithiumhydroxid, Natriumhydroxid und Kaliumhydroxid sowie Alkalimetallalkoxide wie Natriummethylat oder Natriumethylat.

Eine bevorzugte Reaktionstemperatur für das Verfahren der Erfindung beträgt 130 bis 180° C. Besonders bevorzugt sind bei der Herstellung von Anlagerungsprodukten ausschließlich des Ethylenoxids bzw. gemischten Anlagerungsprodukten von Ethylenoxid und Propylenoxid mit endständigen Ethylenoxideinheiten Reaktionstemperaturen bei 150 bis 180° C, wobei in diesen Fällen eine Katalysatorkonzentration von 2 bis 5 Gew.-% ebenfalls besonders bevorzugt ist. Amide bzw. Alkanolamide können bei den beanspruchten Katalysatorkonzentrationen in untergeordnetem Ausmaß umlagern.

Bevorzugte Druckbedingungen für das Verfahren der Erfindung sind 1 bis 10, insbesondere 1 bis 5 bar Überdruck.

Gemäß einer weiteren vorteilhaften Ausführungsform geht man bei dem Verfahren der Erfindung von reaktionsfähige Wasserstoffatome aufweisenden Aminen bzw. Carbonsäureamiden aus, die aus der von Monoalkanolaminen, Dialkanolaminen, Trialkanolaminen, Monoalkyldialkanolaminen, Dialkylmonoalkanolaminen, Monoalkylaminen, Dialkylaminen, Alkylendiaminen, Alkylentriaminen, Alkanolamiden und primären Säureamiden gebildeten Gruppe ausgewählt sind und die dem Fachmann als Ausgangsprodukte für Polyalkoxylierungsverfahren, z.B. zur Herstellung von nichtionischen Tensiden, bekannt sind. Typische Vertreter der einzusetzenden Amine bzw. Carbonsäureamide ergeben sich aus der folgenden Aufstellung:

Primäre und sekundäre Monoamine, vorzugsweise solche mit Siedepunkten über 95° C, z.B.

Amylamin, n-Pentylamin, 3-Methylbutylamin, n-Heptylamin, n-Octylamin, 2-Ethylhexylamin, Isononylamin, Isononyloxypropylamin, Cyclopentylamin, Cyclohexylamin, Furfurylamin, Dodecylamin, Tetradecylamin, Hexadecylamin, Octadecylamin, Eicosylamin, Docosylamin;
weiterhin $C_8$-$C_{22}$-Fettamine in Form ihrer technischen Gemische, wie sie aus synthetischen und/oder natürlichen Fettsäuren der entsprechenden Anzahl der Kohlenstoffatome erhältlich sind, z.B. Cocosfettamin, Oleylamin, Stearylamin, Talgfettamin;
Di-n-propylamin, Di-n-butylamin, Diisopropylamin, Distearylamin, Ditalgfettamin;
Diamine, z.B. Ethylendiamin, Propylendiamin, 1,6-Diaminohexan, Dodecylpropylendiamin, Talgfettpropylendiamin, N,N-Dimethylaminopropylamin;
Polyamine, z.B. Diethylentriamin, Triethylentetramin;
Alkanolamine und Derivate derselben, z.B. Mono-, Di- und Triethanolamin, Aminoethylethanolamin, N-Methyldiethanolamin, N,N-Dimethylethanolamin, Mono-, Di- und Tripropanolamin, N-Hydroxyethylmorpholin;
Primäre Carbonsäureamide, insbesondere von $C_8$-$C_{22}$-Fettsäuren natürlicher und/oder synthetischer Herkunft einschließlich technischer Gemische derselben, z.B. von Caprin-, Capryl-, Laurin-, Myristin-, Palmitin-, Stearin- sowie Kokosfett- und Talgfettsäuren;

Monoalkanolamide und Polyalkanolamide, insbesondere von $C_8$-$C_{22}$-Fettsäuren natürlichen und/oder synthetischen Ursprungs der obigen Aufzählung, z.B. Kokosfettsäure-di- und polydiethanolamid, Talgfettsäurediethanolamid.

Die Erfindung wird im folgenden anhand bevorzugter Ausführungsbeispiele für die Ethoxylierung von Aminen bzw. Amiden erläutert (EO = Ethylenoxid).

Beispiel 1.

Ethoxylierung von Triethanolamin mit 2 Mol EO

Ein 10 l-Edelstahl-Rührautoklav wurde mit 2536 g (17 mol) Triethanolamin und 120,9 g 30 %-iger methanolischer Natriummethylatlösung gefüllt, das Produktgemisch fünfmal mit Stickstoff gespült und zur Entfernung des Methanols 30 Minuten bei 100 °C im Wasserstrahlvakuum evakuiert. Anschließend wurde bei 100 °C ein Stickstoffpartialdruck von 0,5 bar eingestellt, auf 180 °C erhitzt und innerhalb von 5 Stunden im Druckbereich von 3 bis 5 bar 1496 g EO (34 mol) angelagert. Nach einer einstündigen Nachreaktion bei 180 °C wurde auf 75 °C abgekühlt und zur Entfernung von Restmengen EO bei dieser Temperatur 30 Minuten im Wasserstrahlvakuum evakuiert. Man erhielt 3980 g des Ethoxylats in Form einer hellgelben Flüssigkeit.

Beispiel 2.

Ethoxylierung von Kokosamin

Ein 1 l-Edelstahl-Rührautoklav wurde mit 96,5 g Kokosamin (0,5 mol) und 9,5 g 30 %-iger methanolischer Natriummethylatlösung gefüllt, das Produktgemisch fünfmal mit Stickstoff gespült und zur Entfernung des Methanols 30 Minuten bei 100 °C im Wasserstrahlvakuum evakuiert. Anschließend wurde bei 100 °C ein Stickstoffpartialdruck von 0,5 bar eingestellt, auf 150 °C erhitzt und innerhalb von 210 Minuten im Druckbereich von 2 bis 5 bar 220 g (5 mol) EO angelagert. Nach einer einstündigen Nachreaktion bei 150 °C wurde auf 75 °C abgekühlt und zur Entfernung von Restmengen EO bei dieser Temperatur 30 Minuten im Wasserstrahlvakuum evakuiert. Man erhielt 300 g des Ethoxylats in Form eines hellgelben Feststoffs.

Das eingesetzte Kokosamin war ein handelsübliches Produkt mit einem primären Amingehalt von 98 % und einer mittleren Kettenlängenverteilung von 7 % $C_8$, 6 % $C_{10}$, 51 % $C_{12}$, 19 % $C_{14}$, 8 % $C_{16}$ und 9 % $C_{18}$ sowie einer Jodzahl von weniger als 3.

Beispiel 3.

Ethoxylierung von Kokosfettsäurediethanolamid

Ein 1 l-Edelstahl-Rührautoklav wurde mit 145 g Kokosfettsäurediethanolamid (0,5 mol) und 9,6 g 30 %-iger methanolischer Natriummethylatlösung gefüllt, das Produktgemisch fünfmal mit Stickstoff gespült und zur Entfernung des Methanols 30 Minuten bei 100 °C im Wasserstrahlvakuum evakuiert. Anschließend wurde bei 100 °C ein Stickstoffpartialdruck von 0,5 bar eingestellt, auf 150 °C erhitzt und innerhalb von 150 Minuten im Druckbereich von 2 bis 5 bar 176 g (4 mol) EO angelagert. Nach einer einstündigen Nachreaktion bei 150 °C wurde auf 75 °C abgekühlt und zur Entfernung von Restmengen EO bei dieser Temperatur 30 Minuten im Wasserstrahlvakuum evakuiert. Man erhielt 310 g des Ethoxylats in Form einer hellgelben festen Substanz.

Das eingesetzte Kokosfettsäurediethanolamid war ein handelsübliches Produkt mit einem Amidgehalt von mindestens 90 %, hergestellt aus einem Fettsäuregemisch der Kettenlängenverteilung 50 - 62 % $C_{12}$, 19 - 23 % $C_{14}$, 8 - 11 % $C_{16}$ und 9 - 18 % $C_{18}$ (als Ölsäure).

**Ansprüche**

1. Verfahren zur Herstellung von Anlagerungsprodukten von Ethylenoxid und/oder Propylenoxid an reaktionsfähige Wasserstoffatome aufweisende Amine bzw. Amide in molaren Verhältnissen von 1 : 1 bis 20 : 1 durch Umsetzung der Amine bzw. Amide mit Ethylenoxid und/oder Propylenoxid unter erhöhter Temperatur und überatmosphärischem Druck in Abwesenheit von Lösemitteln und in Anwesenheit katalytischer Mengen von Alkalimetallhydroxiden und/oder Alkalimetallalkoxiden, dadurch gekennzeichnet, daß man die Alkalimetallhydroxide und/oder Alkalimetallalkoxide in einer Menge von 1,5 bis 5 Gew.-%, bezogen auf das Endprodukt der Reaktion, einsetzt.

2. Verfahren nach Anspruch 1, dadurch gekennzeichnet, daß man die Anlagerungsprodukte bei Temperaturen von 130 bis 180 °C herstellt.

3. Verfahren nach Anspruch 1 oder 2, dadurch gekennzeichnet, daß man zur Herstellung der ethoxylierten bzw. gemischt ethoxylierten/propoxylierten Anlagerungsprodukte mit endständigen Ethoxylatgruppen die Reaktion bei 150 bis 180°C in Gegenwart von 2 bis 5 Gew.-% der Alkalimetallhydroxide und/oder Alkalimetallalkoxide durchführt.

4. Verfahren nach mindestens einem der Ansprüche 1 bis 3, dadurch gekennzeichnet, daß man bei einem Überdruck von 1 - 10, insbesondere 1 - 5 bar, arbeitet.

5. Verfahren nach mindestens einem der Ansprüche 1 bis 4,dadurch gekennzeichnet, daß man von reaktionsfähige Wasserstoffatome aufweisenden Aminen bzw. Carbonsäureamiden ausgeht, die aus der von Monoalkanolaminen, Dialkanolaminen, Trialkanolaminen, Monoalkyldialkanolaminen, Dialkylmonoalkanolaminen, Monoalkylaminen, Dialkylaminen, Alkylendiaminen, Alkylentriaminen, Alkanolamiden und primären Carbonsäureamiden gebildeten Gruppe ausgewählt sind.